(19) Europäisches Patentamt
European Patent Office
Office européen des brevets

(11) Numéro de publication: **0 321 951 A1**

(12) **DEMANDE DE BREVET EUROPEEN**

(21) Numéro de dépôt: **88121400.1**

(51) Int. Cl.4: **A61K 7/06 , A61K 31/505**

(22) Date de dépôt: **21.12.88**

(30) Priorité: **22.12.87 LU 87091**

(43) Date de publication de la demande:
**28.06.89 Bulletin 89/26**

(84) Etats contractants désignés:
**AT BE CH DE FR GB GR IT LI NL SE**

(71) Demandeur: **L'OREAL**
**14, Rue Royale**
**F-75008 Paris(FR)**

(72) Inventeur: **Grollier, Jean-François**
**16 bis Boulevard Morland**
**F-75004 Paris(FR)**
Inventeur: **Rosenbaum, Georges**
**2 rue J.H. Mansart**
**F-92600 Asnières(FR)**

(74) Mandataire: **Casalonga, Axel et al**
**BUREAU D.A. CASALONGA - JOSSE**
**Morassistrasse 8**
**D-8000 Munich 5(DE)**

(54) **Association de dérivés de pyrimidine et d'agents anti-inflammatoires stéroidiens fluorés pour induire et stimuler la croissance des cheveux et diminuer leur chute.**

(57) Association destinée à induire et à stimuler la croissance des cheveux et diminuer leur chute, caractérisée par le fait qu'elle comprend :

a/ un composant (A) contenant dans un milieu physiologiquement acceptable, au moins un agent anti-inflammatoire stéroïdien fluoré, et

b/ un composant (B) contenant dans un milieu physiologiquement acceptable au moins un dérivé de pyrimidine, répondant à la formule :

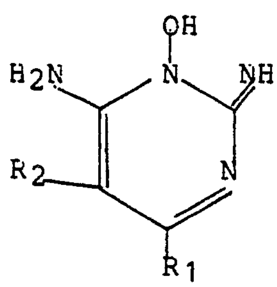

(I)

dans laquelle $R_1$ désigne un groupement

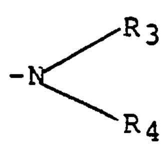

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle,

EP 0 321 951 A1

hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

# Association de dérivés de pyrimidine et d'agents anti-inflammatoires stéroïdiens fluorés pour induire et stimuler la croissance des cheveux et diminuer leur chute.

L'invention est relative à l'association de dérivés de pyrimidine et d'agents anti-inflammatoires stéroïdiens fluorés en vue d'induire et de stimuler la croissance des cheveux et diminuer leur chute.

L'activité des follicules pileux est cyclique. A la phase anagène active, qui dure plusieurs années et au cours de laquelle le cheveu s'allonge, succède une phase de repos (télogène) de quelques mois. A la fin de cette période de repos, le cheveu tombe et un autre cycle recommence.

La chevelure se renouvelle donc en permanence; sur les 100.000 à 150.000 cheveux que comporte une chevelure, à chaque instant 10% environ sont au repos et seront donc remplacés en quelques mois.

Dans presque tous les cas, la chute des cheveux survient chez des sujets prédisposés génétiquement; elle atteint plus particulièrement les hommes.

Cette alopécie est une perturbation du renouvellement capillaire qui entraîne, dans un premier temps, une accélération de la fréquence des cycles aux dépens de la qualité des cheveux puis de leur quantité. Il y a un appauvrissement progressif de la chevelure par régression d'une partie des cheveux dits "terminaux" au stade de "duvets". Des zones sont touchées préférentiellement : golfes temporaux-frontaux, ... chez les hommes; alopécie diffuse du vertex chez les femmes.

Parmi les alopécies, les alopécies acquises, non cicatricielles et diffuses, telle que l'alopécie androgénétique ou androgénique, sont particulièrement difficiles à traiter par opposition aux alopécies localisées telle que la pelade qui serait d'origine immunologique.

On a déjà proposé, dans le passé, le traitement topique de la pelade avec des corticostéroïdes fluorés tels que la fluocinolone, l'halcinonide et la dexaméthasone. Par contre, jusqu'à présent, leur utilisation dans le domaine du traitement de l'alopécie androgénétique ne s'est pas révélée satisfaisante.

On a par ailleurs également utilisé des composés tels que l'amino-6 dihydro-1,2 hydroxy-1 imino-2 pipéridino-4 pyrimidine ou "Minoxidil" dans des compositions permettant de réduire ou de supprimer l'effet de l'alopécie et d'induire et de stimuler la croissance des cheveux et diminuer leur chute.

La demanderesse a découvert maintenant, qu'en associant des agents anti-inflammatoires stéroïdiens avec certains dérivés de pyrimidine, on constatait de façon surprenante, une induction et une stimulation améliorées de la croissance des cheveux et une action plus forte sur le freinage de la chute des cheveux, en particulier dans le cas des alopécies diffuses.

Cette efficacité se caractérise en particulier par une activité supérieure par rapport aux dérivés de pyrimidine utilisés seuls ou par rapport aux agents anti-inflammatoires stéroïdiens.

La demanderesse a également constaté que l'association conforme à l'invention permettait d'obtenir un effet plus rapide que par le passé, ou d'utiliser le dérivé de pyrimidine à une concentration plus faible.

Afin de déterminer l'efficacité ou la rapidité d'action des compositions de l'alopécie, on utilise généralement le trichogramme et plus particulièrement le photo-trichogramme permettant de déterminer entre autre le pourcentage de cheveux en phase anagène par rapport aux cheveux en phase télogène.

L'invention cherche donc essentiellement à augmenter le rapport du nombre de cheveux en phase anagène par rapport au nombre de cheveux en phase télogène.

Un objet de l'invention est donc constitué par l'association d'agents anti-inflammatoires, du type stéroïdien fluoré avec des dérivés de pyrimidine en vue d'induire ou de stimuler la croissance des cheveux et diminuer leur chute.

Un autre objet de l'invention est constitué par une composition cosmétique ou pharmaceutique contenant cette association.

L'invention a également pour objet un dispositif à plusieurs compartiments ou "kits" ou nécessaire, comportant l'association.

D'autres objets de l'invention apparaîtront à la lecture de la description et des exemples qui suivent.

L'association conforme à l'invention est essentiellement caractérisée par le fait qu'elle est constituée par :

a/ un composant (A) comprenant, dans un milieu physiologiquement acceptable, au moins un agent anti-inflammatoire stéroïdien fluoré, et

b/ un composant (B) contenant dans un milieu physiologiquement acceptable, au moins un dérivé de pyrimidine, répondant à la formule :

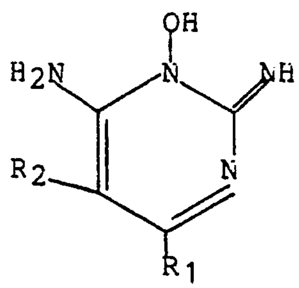

(I)

dans laquelle $R_1$ désigne un groupement

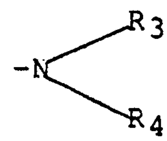

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps en vue d'induire et de stimuler la croissance des cheveux et diminuer leur chute.

Les agents anti-inflammatoires stéroïdiens fluorés sont plus particulièrement choisis parmi :
la dexaméthasone et ses dérivés tels que le 21-acétate, le 21-(3,3-diméthylbutyrate), le 21-phosphate, le 21-tétrahydrophtalate, le 21-diéthylamino-acétate, le 21-isonicotinate, le 21-hémisulfate sodique, l'hémisuccinate, le linoléate, le métasulfobenzoate, le terbutylacétate, le trioxoundécanoate, le valérate;
la betaméthasone et ses dérivés tels que le 21-acétate, le 21-adamantoate, le 17-benzoate, le 17,21-dipropionate, le 21-phosphate disodique, l'hémisuccinate, le valéroacétate, le 21-acétate 17-isobutyrate, le 17-valérate et le 17,21-divalérate;
le 16.17-acétonide de fluocinolone (ou fluocinone);
la fluorométholone;
la triamcinolone et ses dérivés tels que le 16,17-acétonide, l'hexacétonide, le 21-dihydrogèno phosphate sodique 16,17-acétonide, le 16,21-diacétate, le bénétonide, l'hémisuccinate.

Les dérivés sont en particulier des sels, des esters ou des hémiacétals des composés visés.

Conformément à l'invention, des conposés particulièrement préférés sont constitués par les anti-inflammatoires stéroïdiens fluorés, tels que la dexaméthasone, la betaméthasone, le 17-valérate de betaméthasone, l'acétonide de fluocinolone, la fluorométholone et l'acétonide de triamcinolone.

Pour les composés de forn'ule (I), le groupement alkyle ou alcoxy désigne de préférence un groupement ayant 1 à 4 atomes de carbone; le groupement alcényle désigne de préférence un groupement ayant 2 à 5 atomes de carbone; le groupement aryle désigne de préférence un groupement phényle et le groupement cycloalkyle désigne de préférence un groupement ayant 4 à 6 atomes de carbone.

Les conposés particulièrement préférés de formule (I) sont choisis parmi les composés dans lesquels $R_2$ désigne hydrogène et $R_1$ désigne un groupement :

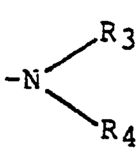

dans lequel $R_3$ et $R_4$ forment un cycle pipéridinyle, ainsi que leurs sels tels que, par exemple, le sulfate.

Parmi ces composés, un composé particulièrement préféré est constitué par l'amino-6 dihydro-1,2 hydroxy-1 imino-2 pipéridino-4 pyrimidine, encore appelé "Minoxidil".

L'agent anti-inflammatoire stéroïdien fluoré est utilisé dans le composant (A) dans des proportions comprises entre 0,01 et 5% en poids, de préférence entre 0,02 et 3%, et plus particulièrement entre 0,02 et 1% en poids; le dérivé de pyrimidine de formule (I) est utilisé de préférence dans le composant (B) dans des proportions comprises entre 0,05 et 10% en poids, de préférence entre 0,05 et 5% en poids et en particulier entre 0,5 et 4% en poids.

Lorsque les composants (A) et (B) sont utilisés dans une composition unique, la proportion en agents anti-inflammatoires stéroïdiens fluorés est comprise entre 0,01 et 3% en poids par rapport au poids total de la composition, de préférence entre 0,01 et 2% et en particulier entre 0,01 et 1%, et le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

Le milieu physiologiquement acceptable pour les composants (A) et (B) est un milieu utilisable en pharmacie et en cosmétique et peut être constitué notamment par de l'eau, un mélange d'eau et d'un ou plusieurs solvants organiques ou par un ou plusieurs solvants organiques cosmétiquement ou pharmaceutiquement acceptables.

Le milieu est de préférence anhydre, c'est-à-dire qu'il contient moins de 1% d'eau, ce milieu étant constitué par un ou plusieurs solvants organiques définis ci-dessus.

Les solvants utilisables sont choisis plus particulièrement parmi les alcools inférieurs en $C_1$-$C_4$ tels que l'alcool éthylique, l'alcool isopropylique, l'alcool tertiobutylique, des alkylèneglycols comme le propylèneglycol, des alkyléthers de mono- et de dialkylèneglycol, et plus particulièrement le monoéthyléther d'éthylèneglycol, le monométhyléther de propylèneglycol et le monoéthyléther de diéthylèneglycol.

Les solvants, lorsqu'ils sont utilisés dans un milieu aqueux, sont présents de préférence dans des proportions comprises entre 1 et 80% en poids par rapport au poids total de la composition.

Les milieux physiologiquement acceptables peuvent être épaissis ou non. On utilise dans ce but des agents épaississants ou gélifiants bien connus dans l'état de la technique, tels que plus particulièrement les hétérobiopolysaccharides tels que la gomme de xanthane ou les scléroglucanes, les dérivés de cellulose, les polymères acryliques réticulés ou non.

Les épaississants sont présents de préférence dans des proportions comprises entre 0,1 et 5% en poids et en particulier entre 0,4 et 3% en poids, par rapport au poids total de chacun des composants lorsqu'ils sont utilisés de façon séparée ou par rapport au poids total de la composition contenant les composants (A) et (B).

Les compositions constituées, soit par les composants (A) et (B) ou par la composition contenant les deux composants (A) et (B), peuvent également contenir tous autres adjuvants habituellement utilisés dans les compositions destinées à une application topique, cosmétique ou pharmaceutique, et plus particulièrement des agents conservateurs, des agents complexants, des colorants, des agents alcalinisants ou acidifiants, des agents tensio-actifs, anioniques, cationiques, non ioniques, amphotères ou leurs mélanges, des polymères anioniques, cationiques, non ioniques ou amphotères, ainsi que leurs mélanges.

Le pH de ces compositions lorsqu'elles contiennent de l'eau peut varier entre 4 et 9.

Ces compositions peuvent également être conditionnées sous pression dans des dispositifs aérosols.

Dans le composant (B), les dérivés de pyrimidine de formule (I) peuvent être présents, soit sous forme dissoute dans le milieu physiologiquement acceptable, ou alors en totalité ou partiellement en suspension dans ce milieu, en particulier sous forme de particules ayant une granulométrie inférieure à 80 microns, de préférence inférieure à 20 microns et en particulier inférieure à 5 microns.

Une première forme de réalisation de l'invention consiste à utiliser l'association définie ci-dessus, sous forme d'une composition contenant les composants (A) et (B).

Une forme particulièrement préférée de l'invention consiste à conserver dans des dispositifs séparés les composants (A) et (B) et à préparer la composition contenant l'agent anti-inflammatoire stéroïdien fluoré et le dérivé de pyrimidine de formule (I) de façon extemporanée tout juste avant application.

Une autre forme de réalisation consiste enfin à appliquer les composants (A) et (B) de façon séparée, soit simultanément, soit de façon successive ou décalée dans le temps.

L'association conforme à l'invention peut être conditionnée dans ces cas, en particulier dans un dispositif à plusieurs compartiments encore appelé "Kit" ou nécessaire, le premier compartiment contient le composant (A) avec l'agent anti-inflammatoire stéroïdien fluoré et le second compartiment contient le composant (B) à base du dérivé de pyrimidine de formule (I). Ce dispositif peut être équipé d'un moyen de mélange des compositions tout juste au moment de l'application.

Le traitement pour induire et stimuler la croissance des cheveux et diminuer leur chute consiste

principalement à appliquer sur les zones alopéciques du cuir chevelu et des cheveux d'un individu, l'association telle que définie ci-dessus, soit au moyen d'une composition unique, soit par application des composants (A) et (B) ou (B) et (A), de façon successive ou décalée dans le temps.

Le mode d'application préféré consiste à appliquer 1 à 2 grammes de la composition ou de chacun des composants (A) et (B) sur la zone alopécique du cuir chevelu à une fréquence de une à deux applications par jour pendant 1 à 7 jours par semaine et ceci pendant une durée de 1 à 6 mois.

La demanderesse a constaté des effets particulièrement significatifs au bout de deux mois de traitement.

Le procédé de traitement présente les caractéristiques d'un traitement thérapeutique dans la mesure où l'association conforme à l'invention a une activité thérapeutique au niveau des mécanismes biologiques du cycle pilaire et des dysfonctionnements de celui-ci.

Un objet de l'invention est donc également l'utilisation de l'association telle que définie ci-dessus pour la préparation d'un médicament ayant pour effet d'induire ou de stimuler la croissance des cheveux et de freiner leur chute.

Le procédé présente, par ailleurs, les caractéristiques d'un procédé cosmétique dans la mesure où il permet d'embellir les cheveux ou le cuir chevelu au sens cosmétique du terme.

Les exemples suivants sont destinés à illustrer l'invention sans pour autant présenter un caractère limitatif.

## EXAMPLE 1

On conditionne en kit deux compositions (A) et (B) renfermant respectivement :

| Composition (A) : | |
|---|---|
| - Dexaméthasone | 0,40 g |
| - Propylèneglycol | 20,00 g |
| - Alcool éthylique | 50,00 g |
| - Eau | qsp 100,00 g |
| **Composition (B) :** | |
| - Minoxidil | 1,00 g |
| - Propylèneglycol | 20,00 g |
| - Alcool éthylique | 50,00 g |
| - Eau | qsp 100,00 g |

On applique sur les parties alopéciques du cuir chevelu le mélange extemporané des deux conpositions (A) et (B).

## EXEMPLE 2

On conditionne en kit deux compositions (A) et (B) renfermant respectivement :

| Composition (A) : | |
|---|---|
| - Bétaméthasone 17-valérate | 0,10 g |
| - Propylèneglycol | 20,00 g |
| - Alcool éthylique | 50,00 g |
| - Eau | qsp 100,00 g |
| Composition (B) : | |
| - Minoxidil | 2,00 g |
| - Alcool éthylique | 95,00 g |
| - Propylèneglycol | qsp 100,00 g |

On applique sur les parties alopéciques du cuir chevelu en succession immédiate les deux compositions (A) puis (B).

EXEMPLE 3

On prépare la composition suivante :

| | |
|---|---|
| - Acétonide de fluocinolone | 0,025 g |
| - Minoxidil | 1,00 g |
| - Alcool éthylique | 50,00 g |
| - Propylèneglycol | 20,00 g |
| - Hydroxypropyl cellulose vendue par la Société HERCULES sous la dénomination "KLUCEL G" | 3,00 g |
| - Eau | qsp 100,00 g |

On applique sur les parties alopéciques du cuir chevelu la composition ci-dessus à raison d'une application journalière d'environ 4 mg par $cm^2$ de cuir chevelu, pendant 2 mois.

EXEMPLE 4

On conditionne en kit deux compositions (A) et (B) renfermant respectivement :

| Composition (A) : | |
|---|---|
| - Fluorométholone | 0,10 g |
| - Propylèneglycol | 20,00 g |
| - Alcool éthylique | 50,00 g |
| - Eau | qsp 100,00 g |
| Composition (B) : | |
| - Minoxidil | 2,50 g |
| - Propylèneglycol | 20,00 g |
| - Alcool éthylique | 50,00 g |
| - Gomme de xanthane vendue par la Société KELCO sous la dénomination "KELTROL T" | 0,75 g |
| - Eau | qsp 100,00 g |

On applique sur les parties alopéciques du cuir chevelu les compositions (A) puis (B) en succession décalée dans le temps : (A) le matin et (B) le soir.

EXEMPLE 5

On conditionne en kit deux compositions (A) et (B) renfermant respectivement :

| Composition (A) : | |
|---|---|
| - Acétonide de triamcinolone | 0,10 g |
| - Propylèneglycol | 20,00 g |
| - Alcool éthylique | 50,00 g |
| - Hydroxypropyl cellulose vendue par la Société HERCULES sous la dénomination "KLUCEL G" | 2,00 g |
| - Eau | qsp 100,00 g |
| **Composition (B) :** | |
| - Minoxidil | 1,50 g |
| - Propylèneglycol | 20,00 g |
| - Alcool éthylique | 50,00 g |
| - Eau | qsp 100,00 g |

On applique sur les parties alopéciques du cuir chevelu les compositions en alternance journalière, premier jour : la composition ((A), second jour : la composition (B).

EXEMPLE 6

| | |
|---|---|
| - Dexaméthasone | 2,4 g |
| - Minoxidil | 1,5 g |
| - Propylèneglycol distillé | 6,45 g |
| - Alcool éthylique absolu | qsp 100,00 g |

EXEMPLE 7

| | |
|---|---|
| - 16,17-acétonide de fluocinolone | 3,0 g |
| - Minoxidil | 1,5 g |
| - Propylène glycol distillé | 6,45 g |
| - Alcool éthylique absolu | qsp 100,00 g |

EXEMPLE 8

| | |
|---|---|
| - Fluorométholone | 0,3 g |
| - Minoxidil | 2,00 g |
| - Propylèneglycol distillé | 6,45 g |
| - Alcool éthylique absolu | qsp 100,00 g |

## EXEMPLE 9

On conditionne en kit deux compositions (A) et (B) renfermant respectivement :

| Composition (A) : | |
|---|---|
| - 16, 17-acétonide de fluocinolone<br>- Propylèneglycol distillé<br>- Alcool éthylique absolu | 5,00 g<br>6,45 g<br>qsp 100,00 g |
| Composition (B) : | |
| - Minoxidil<br>- Propylèneglycol distillé<br>- Alcool éthylique absolu | 2,50 g<br>6,45 g<br>qsp 100,00 g |

dont on mélange 60% en poids de la composition (A) avec 40% en poids de la composition (B) avant l'application.

## EXEMPLE 10

On conditionne en kit deux compositions (A) et (B) renfermant respectivement :

| Composition (A) : | |
|---|---|
| - 17-valérate de bétaméthasone<br>- Propylèneglycol distillé<br>- Alcool éthylique absolu | 3,8 g<br>6,45 g<br>qsp 100,00 g |
| Composition (B) : | |
| - Minoxidil<br>- Propylèneglycol distillé<br>- Alcool éthylique absolu | 2,50 g<br>6,45 g<br>qsp 100,00 g |

On applique, sur les zones alopéciques du cuir chevelu, les compositions (A) et (B) en succession décalée dans le temps, en alternance journalière :
premier jour : application de la composition (A)
second jour : application de la composition (B)

## EXEMPLE 11

| Composition (A) : | |
|---|---|
| - Dexaméthasone<br>- Propylèneglycol distillé<br>- Alcool éthylique absolu | 2,4 g<br>6,45 g<br>qsp 100,00 g |
| Composition (B) : | |
| - Minoxidil<br>- Propylèneglycol distillé<br>- Alcool éthylique absolu | 2,50 g<br>6,45 g<br>qsp 100,00 g |

dont on mélange 25% en poids de la composition (A) avec 75% en poids de la composition (B) avant l'application.

## Revendications

1. Association destinée à induire et à stimuler la croissance des cheveux et diminuer leur chute, caractérisée par le fait qu'elle comprend :

a/ un composant (A) contenant dans un milieu physiologiquement acceptable, au moins un agent anti-inflammatoire stéroïdien fluoré, et

b/ un composant (B) contenant dans un milieu physiologiquement acceptable au moins un dérivé de pyrimidine, répondant à la formule :

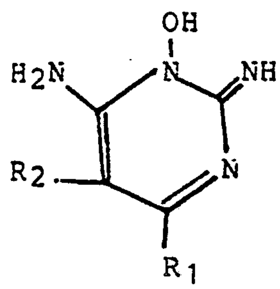

(I)

dans laquelle $R_1$ désigne un groupement

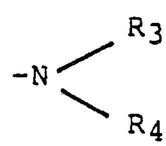

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

2. Association selon la revendication 1, caractérisée par le fait que l'agent anti-inflammatoire stéroïdien fluoré est choisi parmi la dexaméthasone et ses dérivés, la betaméthasone et ses dérivés, le 16,17-acétonide de fluocinolone, la fluorométholone, la triamcinolone.

3. Association selon la revendication 2, caractérisée par le fait que les dérivés de dexaméthasone sont le 21-acétate, le 21-(3,3-diméthylbutyrate), le 21-phosphate, le 21-tétrahydrophtalate, le 21-diéthylamino acétate, le 21-isonicotinate, le 21-hémisulfate sodique, l'hémisuccinate, le linoléate, le métasulfobenzoate, le terbutylacétate, le trioxoundécanoate, le valérate; que les dérivés de betaméthasone sont choisis parmi le 21-acétate, le 21-adamantoate, le 17-benzoate, le 17,21-dipropionate, le 21-phosphate disodique, l'hémisuccinate, le valéroacétate, le 21-acétate 17-isobutyrate, le 17-valérate et le 17,21-divalérate; que les dérivés de triamcinolone sont choisis parmi le 16,17-acétonide, l'hexacétonide, le 21-dihydrogénophosphate sodique 16,17-acétonide, le 16,21-diacétate, le bénétonide, l'hémisuccinate.

4. Association selon l'une quelconque des revendications 1 à 3, caractérisée par le fait que le dérivé de pyrimidine de formule (I) est l'amino-6 dihydro-1,2 hydroxy-1 imino-2 pipéridino-4 pyrimidine ou "Minoxidil".

5. Association selon l'une quelconque des revendications 1 à 4, caractérisée par le fait que l'agent anti-inflammatoire stéroïdien fluoré est présent dans le composant (A) dans des proportions comprises entre 0,01 et 5% en poids, de préférence entre 0,02 et 3% et en particulier entre 0,02 et 1% en poids, et que le dérivé de pyrimidine est présent dans le composant (B) dans des proportions comprises entre 0,05 et 10% en poids, de préférence entre 0,05 et 5% en poids et en particulier entre 0,5 et 4% en poids.

6. Association selon l'une quelconque des revendications 1 à 5, caractérisée par le fait que l'association est présente dans une composition unique, dans laquelle l'agent anti-inflammatoire stéroïdien fluoré est présent dans des proportions comprises entre 0,01 et 3% en poids par rapport au poids total de la composition, de préférence entre 0,01 et 2% et en particulier entre 0,01 et 1%, et que le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

7. Association selon l'une quelconque des revendications 1 à 6, caractérisée par le fait que le milieu physiologiquement acceptable pour les composants (A) et (B) est un milieu constitué par de l'eau ou un mélange d'eau et d'un ou plusieurs solvants organiques ou par un ou plusieurs solvants organiques acceptables en cosmétique ou en pharmacie.

8. Association selon l'une quelconque des revendications 1 à 6, caractérisée par le fait que le milieu physiologiquement acceptable est anhydre.

9. Association selon l'une quelconque des revendications 1 à 8, caractérisée par le fait que l'un au moins des composants contient un solvant choisi parmi les alcools inférieurs en $C_1$-$C_4$, les alkylène-glycols, les alkyléthers de mono- et de dialkylène-glycol.

10. Association selon l'une quelconque des revendications 1 à 9, caractérisée par le fait que l'un au moins des composants (A) ou (B) est épaissi au moyen d'agents épaississants ou gélifiants.

11. Association selon l'une quelconque des revendications 1 à 10, caractérisée par le fait que l'un au moins des composants (A) ou (B) contient également des adjuvants cosmétiquement ou pharmaceutiquement acceptables, choisis parmi des agents conservateurs, des agents complexants, des colorants, des agents alcalinisants ou acidifiants, des agents tensio-actifs, anioniques, cationiques, non ioniques ou amphotères, ainsi que leurs mélanges, des polymères anioniques, cationiques, non ioniques ou amphotères, ainsi que leurs mélanges.

12. Procédé de traitement cosmétique des cheveux ou du cuir chevelu, caractérisé par le fait que l'on applique les composants (A) et (B) de l'association telle que définie dans l'une quelconque des revendications 1 à 11, au moyen d'une composition unique ou de façon séparée, soit simultanément, soit de façon successive ou décalée dans le temps, sur les cheveux ou le cuir chevelu.

13. Dispositif à plusieurs compartiments ou "kit", caractérisé par le fait qu'il comprend dans un premier compartiment un composant (A) tel que défini dans l'une quelconque des revendications 1 à 11, et dans un second compartiment le composant (B) tel que défini également dans l'une quelconque des revendications 1 à 11.

14. Association selon l'une quelconque des revendications 1 à 11, pour son application comme médicament pour le traitement thérapeutique de la chute des cheveux.

15. Utilisation de l'association selon l'une quelconque des revendications 1 à 11, pour la préparation d'un médicament destiné à être utilisé dans le traitement thérapeutique de l'alopécie androgénétique.

Office européen des brevets

# RAPPORT DE RECHERCHE EUROPEENNE

Numero de la demande

EP 88 12 1400

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | S.T.N. FILE SUPPLIER, Karlsruhe, DE * Résumé * & CHEMICAL ABSTRACTS, vol. 106, 1987, page 339, no. 201544m, & PATENT ABSTRACTS OF JAPAN, vol. 11, no. 214 (C-434)[2661], 10 Juillet 1987; & JP-A-62 33 111 (MICHIO NAKANISHI) 13-02-1987 | 1,2,4-12,13-15 | A 61 K 7/06<br>A 61 K 31/505 |
| Y | PATENT ABSTRACTS OF JAPAN, vol. 11, no. 115 (C-415)[2562], page 145 C 415; & JP-A-61 260 010 (YAMAGUCHI YAKUHIN SHOKAI K.K.) 18-11-1986 * Résumé * | 1,2,4-12,13-15 | |
| Y | FR-A-2 310 770 (DSO "PHARMACHIM") * En entier * | 1,2,4-12,13-15 | |
| A | PATENT ABSTRACTS OF JAPAN, vol. 11, no. 3 (C-395)[2450], page 107 C 395; & JP-A-61 183 209 (LION CORP.) 15-08-1986 * Résumé * | 1,2 | **DOMAINES TECHNIQUES RECHERCHES (Int. Cl.4)** |
| A,P | WO-A-8 807 361 (UPJOHN) * En entier * | 1-15 | A 61 K |
| A | WO-A-8 505 270 (MORTIMER) * Page 17, exemple: "Preparation 1 plus vasodilator" * | 1-15 | |

Le présent rapport a été établi pour toutes les revendications

| Lieu de la recherche | Date d'achèvement de la recherche | Examinateur |
|---|---|---|
| LA HAYE | 04-04-1989 | FISCHER J.P. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)